# NEW EUROPEAN PATENT SPECIFICATION

(11) **EP 3 735 217 B2**
(45) Date of publication and mention of the opposition decision: **28.10.2026**
(45) Mention of the grant of the patent: 09.08.2023
(21) Application number: 18826711.6
(22) Date of filing: 21.12.2018
(51) Int. Cl.: A61K 8/44, A61K 8/46, A61Q 5/02, A61K 8/73, A61K 8/81, A61K 8/92

(54) **COMPOSITION COMPRISING SURFACTANTS, CATIONIC POLYMERS AND A PLANT OIL**
ZUBEREITUNG ENTHALTEND TENSIDEN, KATIONISCHE POLYMEREN UND EIN PFLANZENÖL
COMPOSITION COMPRENANT DES TENSIOACTIFS, DES POLYMÈRES CATIONIQUES ET UNE HUILE VÉGÉTALE

(30) Priority: 04.01.2018 FR 1850043
(43) Date of publication of application: 11.11.2020
(73) Proprietor: L'OREAL, 75008 Paris (FR)
(72) Inventor: MODESTE, Véronique, 93400 SAINT OUEN (FR); D'ARRAS, Marie-Florence, 93400 SAINT OUEN (FR); JIA, Haidong, Shanghai 201206 (CN)
(74) Representative: Casalonga
(86) International application number: PCT/EP2018/086561
(87) International publication number: WO 2019/134855

(56) References cited:
- WO-A1-2016/101138
- DE-A1- 10 354 116
- US-A- 5 344 643
- US-A- 5 883 059
- US-A- 6 110 451
- DATABASE GNPD [online] MINTEL; November 2017 (2017-11-01), "Shampoo", XP002784025, Database accession no. 5285001

## Description

The present invention relates to a composition comprising at least one anionic surfactant, at least one amphoteric or zwitterionic surfactant, at least one cationic polysaccharide, at least one alkyldiallylamine polymer and/or at least one dialkyldiallylammonium polymer, at least one plant oil and at least one fatty ether.

The invention also relates to a process for treating keratin fibres, comprising at least one step of applying a composition according to the invention to said keratin fibres.

It is well known that hair may be sensitized or embrittled to varying degrees as a result of the action of atmospheric agents such as light, water and moisture, and also repeated mechanical or chemical treatments such as brushing, combing, washing, bleaching, permanent waving, relaxing and/or dyeing. These attacking factors impair the hair fibre and reduce its mechanical properties such as the tensile strength, the breaking load and the elasticity, or its resistance to swelling in an aqueous medium. The hair is dull, coarse and brittle. The hair is difficult to disentangle and to style.

Substances for protecting the hair against such degradation have been sought for many years in the cosmetics industry; products that improve the cosmetic properties, especially the disentangling, soft feel and volume of the head of hair, and that preserve or reinforce the intrinsic mechanical properties of keratin fibres, such as the tensile strength, the breaking load and the elasticity, or their resistance to swelling in an aqueous medium, are sought in particular.

Thus, shampoos have been proposed, especially for sensitized hair, which combine a cationic polymer and a silicone, to obtain acceptable cosmetic performance qualities.

However, these compositions have several drawbacks: presence of silicone, the environmental profile (biodegradability, water footprint) of which is not always optimal, generally opaque (sparingly aesthetic) appearance of the shampoo associated with the presence of silicone, start of foaming and foam quality that are judged as being insufficient, and rapid regreasing of the hair accompanied by lankness.

In addition, repeated applications of these compositions often have the effect of giving the hair an unpleasant feel, loss of volume and liveliness of the head of hair, and occasionally lack of sheen.

The interest in developing a washing composition that is advantageously silicone-free, having improved working qualities and good cosmetic properties, and that is capable of washing keratin fibres without making them lank so as to give good conditioning properties to keratin fibres, in particular to sensitized, embrittled or damaged keratin fibres, and also more particularly to fine hair, arose from these observations.

These aims are achieved by the present invention, the subject of which is especially a composition comprising:
(i) at least one anionic surfactant,
(ii) at least one amphoteric or zwitterionic surfactant,
(iii) at least one cationic polymer chosen from cationic polysaccharides,
(iv) at least one cationic polymer chosen from alkyldiallylamine polymers, dialkyldiallylammonium polymers, and mixtures thereof, and
(v) one or more plant oils, the total content of which is greater than or equal to 0.1% by weight relative to the total weight of the composition; and
(vi) at least one fatty ether chosen from the compounds of formula (4) below:

   R-O-R' (4)
in which:
R and R', which may be identical or different, represent a linear or branched C₆-C₂₅ alkyl or alkenyl radical;
it being understood that the weight ratio between the total content of cationic polysaccharide(s) (iii), on the one hand, and the total content of cationic polymer(s) (iv), on the other hand, is greater than or equal to 1.0.

It has been found that the composition according to the invention has good working qualities, especially good foam quality.

It has also been found that hair treated with the composition according to the invention is particularly clean and has good cosmetic properties. Hair thus treated is particularly light, soft- and smooth-feeling, shiny, easy to disentangle, more manageable, and has good volume and also repaired ends.

Furthermore, it has been observed that the composition according to the invention gives the hair strength.

Moreover, the Applicant has also found that the use of the composition according to the invention makes it possible to substantially reduce its environmental impact relative to a conventional washing and conditioning composition.

A subject of the invention is also a process for the treatment of, preferably for washing, keratin fibres, in particular human keratin fibres such as the hair, comprising at least one step of applying to said keratin fibres a composition according to the invention.

Other subjects, characteristics, aspects and advantages of the invention will emerge even more clearly on reading the description and the example that follows.

In the present description, and unless otherwise indicated:
- the expression "at least one" is equivalent to the expression "one or more" and can be replaced therewith;
- the expression "between" is equivalent to the expression "ranging from" and can be replaced therewith, and implies that the limits are included;
- according to the present application, the term "keratin fibres" denotes human keratin fibres and more particularly the hair;
- the term "cationic polymer" means any polymer containing cationic group(s) and/or group(s) which can be ionized to cationic group(s), and preferably not containing anionic group(s) and/or group(s) which can be ionized to anionic group(s). Preferably, the cationic polymer is hydrophilic or amphiphilic. Preferably, the cationic polymers that may be used according to the invention are non-silicone; in other words, the cationic polymers that may be used according to the invention do not comprise any silicon atoms.

Preferably, the composition according to the invention is silicone-free.

The term "silicone-free" means that the composition according to the invention does not comprise any silicone, or that the silicone(s) present in the composition according to the invention are included in a total content of less than or equal to 0.1% by weight, preferably less than 0.05% relative to the total weight of the composition according to the invention, and better still is free of silicone (0%).

The term "silicone" means any organosilicon polymer or oligomer of linear or cyclic and branched or crosslinked structure, of variable molecular weight, obtained by polymerization and/or polycondensation of suitably functionalized silanes and essentially constituted of a repetition of main units in which the silicon atoms are connected to each other via oxygen atoms (siloxane bond -Si-O-Si-), optionally substituted hydrocarbon-based radicals being connected directly to said silicon atoms via a carbon atom; and more particularly dialkylsiloxane polymers, amino silicones and dimethiconols.

### Anionic surfactants

The composition according to the present invention comprises at least one anionic surfactant.

The term "anionic surfactant" means a surfactant comprising, as ionic or ionizable groups, only anionic groups.

In the present description, a species is termed "anionic" when it bears at least one permanent negative charge or when it can be ionized into a negatively charged species, under the conditions of use of the composition of the invention (for example the medium or the pH) and not comprising any cationic charge.

The anionic surfactants may be sulfate, sulfonate and/or carboxylic (or carboxylate) surfactants. Needless to say, a mixture of these surfactants may be used.

It is understood in the present description that:
- the carboxylate anionic surfactants comprise at least one carboxylic or carboxylate function (-COOH or -COO⁻) and may optionally also comprise one or more sulfate and/or sulfonate functions;
- the sulfonate anionic surfactants comprise at least one sulfonate function (-SO₃H or -SO₃⁻) and may optionally also comprise one or more sulfate functions, but do not comprise any carboxylate functions; and
- the sulfate anionic surfactants comprise at least one sulfate function but do not comprise any carboxylate or sulfonate functions.

The carboxylic anionic surfactants that may be used thus comprise at least one carboxylic or carboxylate function (-COOH or -COO⁻).

They may be chosen from the following compounds: acylglycinates, acyllactylates, acylsarcosinates, acylglutamates; alkyl-D-galactosideuronic acids, alkyl ether carboxylic acids, alkyl(C₆-C₃₀ aryl) ether carboxylic acids, alkylamido ether carboxylic acids; and also the salts of these compounds;
the alkyl and/or acyl groups of these compounds comprising from 6 to 30 carbon atoms, especially from 12 to 28, better still from 14 to 24 or even from 16 to 22 carbon atoms; the aryl group preferably denoting a phenyl or benzyl group;
these compounds possibly being polyoxyalkylenated, in particular polyoxyethylenated, and then preferably comprising from 1 to 50 ethylene oxide units and better still from 2 to 10 ethylene oxide units.

Use may also be made of C₆-C₂₄ alkyl monoesters of polyglycoside-polycarboxylic acids such as C₆-C₂₄ alkyl polyglycoside-citrates, C₆-C₂₄ alkyl polyglycoside-tartrates and C₆-C₂₄ alkyl polyglycoside-sulfosuccinates, and salts thereof.

Among the above carboxylic surfactants, mention may be made most particularly of polyoxyalkylenated alkyl(amido) ether carboxylic acids and salts thereof, in particular those including from 2 to 50 alkylene oxide and in particular ethylene oxide groups, such as the compounds sold by the company Kao under the Akypo names.

The polyoxyalkylenated alkyl(amido) ether carboxylic acids that may be used are preferably chosen from those of formula (1):

R₁-(OC₂H₄)ₙ-OCH₂COOA (1)

in which:
- Ri represents a linear or branched C₆-C₂₄ alkyl or alkenyl radical, a (C₈-C₉)alkylphenyl radical, a radical R₂CONH-CH₂-CH₂- with R₂ denoting a linear or branched C₉-C₂₁ alkyl or alkenyl radical;
   preferably, R₁ is a C₈-C₂₀ and preferably C₈-C₁₈ alkyl radical, and aryl preferably denotes phenyl,
- n is an integer or decimal number (average value) ranging from 2 to 24 and preferably from 2 to 10,
- A denotes H, ammonium, Na, K, Li, Mg or a monoethanolamine or triethanolamine residue.

Use may also be made of mixtures of compounds of formula (1), in particular mixtures of compounds bearing different groups R₁.

The polyoxyalkylenated alkyl(amido) ether carboxylic acids that are particularly preferred are those of formula (1) in which:
- R₁ denotes a C₁₂-C₁₄ alkyl, cocoyl, oleyl, nonylphenyl or octylphenyl radical,
- A denotes a hydrogen or sodium atom, and
- n ranges from 2 to 20, preferably from 2 to 10.

Even more preferentially, use is made of the compounds of formula (1) in which R₁ denotes a C₁₂ alkyl radical, A denotes a hydrogen or sodium atom and n ranges from 2 to 10.

Preferentially, the carboxylic anionic surfactants are chosen, alone or as a mixture, from:
- acylglutamates, in particular of C₆-C₂₄ or even C₁₂-C₂₀, such as stearoylglutamates, and in particular disodium stearoylglutamate;
- acylsarcosinates, in particular of C₆-C₂₄ or even C₁₂-C₂₀, such as palmitoylsarcosinates, and in particular sodium palmitoylsarcosinate;
- acyllactylates, in particular of C₁₂-C₂₈ or even C₁₄-C₂₄, such as behenoyllactylates, and in particular sodium behenoyllactylate;
- C₆ -C₂₄ and especially C₁₂ -C₂₀ acylglycinates;
- (C₆-C₂₄)alkyl ether carboxylates, and especially (C₁₂-C₂₀)alkyl ether carboxylates;
- polyoxyalkylenated (C₆-C₂₄)alkyl(amido) ether carboxylic acids, in particular those comprising from 2 to 50 ethylene oxide groups;
in particular in the form of alkali metal or alkaline-earth metal, ammonium or amino alcohol salts.

The sulfonate anionic surfactants that may be used comprise at least one sulfonate function (-SO₃H or -SO₃⁻).

They may be chosen from the following compounds: alkylsulfonates, alkylamidesulfonates, alkylarylsulfonates, α-olefin sulfonates, paraffin sulfonates, alkyl sulfosuccinates, alkyl ether sulfosuccinates, alkylamidesulfosuccinates, alkylsulfoacetates, N-acyltaurates, acylisethionates; alkylsulfolaurates; and also the salts of these compounds;
the alkyl groups of these compounds comprising from 6 to 30 carbon atoms, in particular from 12 to 28, better still from 14 to 24 or even from 16 to 22 carbon atoms; the aryl group preferably denoting a phenyl or benzyl group;
these compounds possibly being polyoxyalkylenated, in particular polyoxyethylenated, and then preferably comprising from 1 to 50 ethylene oxide units and better still from 2 to 10 ethylene oxide units.

Preferentially, the sulfonate anionic surfactants are chosen, alone or as a mixture, from:
- C₆-C₂₄ and especially C₁₂-C₂₀ alkyl sulfosuccinates, especially lauryl sulfosuccinates;
- C₆-C₂₄ and especially C₁₂-C₂₀ alkyl ether sulfosuccinates;
- (C₆-C₂₄)acylisethionates, preferably (C₁₂-C₁₈)acylisethionates;
in particular in the form of alkali metal or alkaline-earth metal, ammonium or amino alcohol salts.

The sulfate anionic surfactants that may be used comprise at least one sulfate function (-OSO₃H or -OSO₃⁻).

They may be chosen from the following compounds: alkyl sulfates, alkyl ether sulfates, alkylamido ether sulfates, alkylaryl polyether sulfates, monoglyceride sulfates; and also the salts of these compounds;
the alkyl groups of these compounds including from 6 to 30 carbon atoms, in particular from 12 to 28, better still from 14 to 24 or even from 16 to 22 carbon atoms; the aryl group preferably denoting a phenyl or benzyl group;
these compounds possibly being (poly)oxyalkylenated, especially (poly)oxyethylenated, and then preferably including from 1 to 50 ethylene oxide units and better still from 1 to 10 ethylene oxide units.

Preferentially, the sulfate anionic surfactants are chosen, alone or as a mixture, from:
- alkyl sulfates, especially C₆-C₂₄ or even C₁₂-C₂₀ alkyl sulfates;
- alkyl ether sulfates, especially C₆-C₂₄ or even C₁₂-C₂₀ alkyl ether sulfates, preferably comprising from 1 to 20 ethylene oxide units;
in particular in the form of alkali metal or alkaline-earth metal, ammonium or amino alcohol salts.

When the anionic surfactant is in salt form, said salt may be chosen from alkali metal salts, such as the sodium or potassium salt, ammonium salts, amine salts and in particular amino alcohol salts, and alkaline-earth metal salts, such as the magnesium salt.

Examples of amino alcohol salts that may be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts and in particular the sodium or magnesium salts are preferably used.

Preferentially, the anionic surfactant(s) are chosen from:
- C₆-C₂₄ and especially C₁₂-C₂₀ alkyl sulfates;
- C₆-C₂₄ and especially C₁₂-C₂₀ alkyl ether sulfates; preferably comprising from 1 to 20 ethylene oxide units;
- C₆-C₂₄ and especially C₁₂-C₂₀ alkyl sulfosuccinates, especially lauryl sulfosuccinates;
- C₆-C₂₄ and especially C₁₂-C₂₀ alkyl ether sulfosuccinates;
- (C₆-C₂₄)acylisethionates, preferably (C₁₂-C₁₈)acylisethionates;
- C₆-C₂₄ and especially C₁₂-C₂₀ acylsarcosinates; especially palmitoylsarcosinates;
- (C₆-C₂₄)alkyl ether carboxylates, preferably (C₁₂-C₂₀)alkyl ether carboxylates;
- polyoxyalkylenated (C₆-C₂₄)alkyl(amido) ether carboxylic acids and salts thereof, in particular those including from 2 to 50 alkylene oxide and in particular ethylene oxide groups;
- C₆-C₂₄ and especially C₁₂-C₂₀ acylglutamates;
- C₆-C₂₄ and especially C₁₂ -C₂₀ acylglycinates;
- and also salts thereof, in particular the alkali metal or alkaline-earth metal or zinc, ammonium or amino alcohol salts thereof;
- and mixtures thereof.

According to a preferred embodiment of the invention, the composition comprises at least one anionic surfactant chosen from C₆-C₂₄ and especially C₁₂-C₂₀ alkyl sulfates, C₆-C₂₄ and especially C₁₂-C₂₀ alkyl ether sulfates, preferably comprising from 1 to 20 ethylene oxide units, polyoxyalkylenated (C₆-C₂₄)alkyl(amido) ether carboxylic acids, in particular those including from 2 to 50 alkylene oxide and more particularly ethylene oxide groups; and salts thereof, especially the alkali metal or alkaline-earth metal, ammonium or amino alcohol salts thereof; and mixtures thereof.

Preferably, the total content of the anionic surfactant(s) present in the composition is between 0.1% and 30% by weight, more preferentially between 1% and 20% by weight, and even more preferentially between 5% and 15% by weight, relative to the total weight of the composition.

### Amphoteric or zwitterionic surfactants

The composition according to the present invention comprises at least one amphoteric or zwitterionic surfactant.

In particular, the amphoteric or zwitterionic surfactant(s) may be optionally quaternized secondary or tertiary aliphatic amine derivatives, in which the aliphatic group is a linear or branched chain including from 8 to 22 carbon atoms, said amine derivatives containing at least one anionic group, for instance a carboxylate, sulfonate, sulfate, phosphate or phosphonate group.

Mention may in particular be made of (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines.

Among the optionally quaternized derivatives of secondary or tertiary aliphatic amines that may be used, as defined above, mention may also be made of the compounds having the respective formulae (3) and (4) below:

Rₐ-CONHCH₂CH₂-N⁺(R_{b})(R_{c})-CH₂COO⁻, M⁺, X⁻ (3)

in which:
- Rₐ represents a C₁₀ to C₃₀ alkyl or alkenyl group derived from an acid RₐCOOH preferably present in hydrolysed coconut kernel oil, or a heptyl, nonyl or undecyl group;
- R_{b} represents a β-hydroxyethyl group; and
- R_{c} represents a carboxymethyl group;
- M⁺ represents a cationic counterion derived from an alkali metal or alkaline-earth metal, such as sodium, an ammonium ion or an ion derived from an organic amine; and
- X⁻ represents an organic or mineral anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;

   R_{a'}-CONHCH₂CH₂-N(B)(B') (4)

   in which:
   - B represents the group -CH₂CH₂OX';
   - B' represents the group -(CH₂)_{z}Y', with z = 1 or 2;
   - X' represents the group -CH₂COOH, -CH₂-COOZ', -CH₂CH₂COOH or CH₂CH₂-COOZ', or a hydrogen atom;
   - Y' represents the group -COOH, -COOZ' or -CH₂CH(OH)SO₃H or the group CH₂CH(OH)SO₃-Z';
   - Z' represents a cationic counterion derived from an alkali metal or alkaline-earth metal, such as sodium, an ammonium ion or an ion derived from an organic amine;
   - R_{a'} represents a C₁₀ to C₃₀ alkyl or alkenyl group of an acid R_{a'}-COOH which is preferably present in coconut kernel oil or in hydrolysed linseed oil, or an alkyl group, especially a C₁₇ group, and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Use may also be made of the compounds of formula (5):

R_{a"}-NHCH(Y")-(CH₂)ₙCONH(CH₂)_{n'}-N(R_{d})(Rₑ) (5)

in which:
- Y" represents the group -COOH, -COOZ" or -CH₂-CH(OH)SO₃H or the group CH₂CH(OH)SO₃-Z";
- R_{d} and Rₑ represent, independently of each other, a C₁ to C₄ alkyl or hydroxyalkyl radical;
- Z" represents a cationic counterion derived from an alkali metal or alkaline-earth metal, such as sodium, an ammonium ion or an ion derived from an organic amine;
- R_{a"} represents a C₁₀ to C₃₀ alkyl or alkenyl group of an acid R_{a"}-COOH which is preferably present in coconut kernel oil or in hydrolysed linseed oil;
- n and n' denote, independently of each other, an integer ranging from 1 to 3.

Among the compounds of formula (5), mention may be made of the compound classified in the CTFA dictionary under the name sodium diethylaminopropyl cocoaspartamide and sold by the company Chimex under the name Chimexane HB.

These compounds may be used alone or as mixtures.

Preferably, the amphoteric surfactant(s) (iv) are chosen, alone or as a mixture, from (C₈-C₂₀)alkylbetaines, (C₈-C₂₀)alkylsulfobetaines, (C₈-C₂₀)alkylamido(C₃-C₈)alkylbetaines and (C₈-C₂₀)alkylamido(C₆-C₈)alkylsulfobetaines, and also the compounds of formulae (3), (4) and (5) as defined previously.

Preferentially, the amphoteric or zwitterionic surfactant(s) are chosen, alone or as a mixture, from (C₈-C₂₀)alkylbetaines such as cocoylbetaine, (C₈-C₂₀)alkylamido(C₃-C₈)alkylbetaines such as cocamidopropylbetaine, and the compounds of formula (5) such as the sodium salt of diethylaminopropyl laurylaminosuccinamate (INCI name: sodium diethylaminopropyl cocoaspartamide).

More preferentially, the amphoteric or zwitterionic surfactant(s) are chosen from (C₈-C₂₀)alkylbetaines such as cocoylbetaine, (C₈-C₂₀)alkylamido(C₃-C₈)alkylbetaines such as cocamidopropylbetaine, and mixtures thereof.

Preferably, the amphoteric or zwitterionic surfactant(s) are present in the composition according to the invention in a total content ranging from 0.1% to 20% by weight, more preferentially in a content ranging from 0.5% to 15% by weight and better still from 1% to 10% by weight relative to the total weight of the composition.

According to a preferred embodiment of the invention, the weight ratio between the total content of anionic surfactant(s), on the one hand, and the total content of amphoteric or zwitterionic surfactant(s), on the other hand, is between 1.0 and 4.0, more preferentially between 1.0 and 3.5 and even more preferentially between 1.5 and 3.0.

### The cationic polysaccharides (iii)

The composition according to the present invention comprises at least one cationic polymer chosen from cationic polysaccharides.

The cationic polysaccharides that may be used preferably have a weight-average molar mass (Mw) of between 500 and 5×10⁶ approximately and preferably between 10³ and 3×10⁶ approximately.

Preferably, the cationic polysaccharide(s) are chosen from cationic celluloses and cationic galactomannan gums.

Examples that may be mentioned more particularly include cellulose ether derivatives including quaternary ammonium groups, cationic cellulose copolymers or cellulose derivatives grafted with a water-soluble quaternary ammonium monomer and cationic galactomannan gums.

The cellulose ether derivatives including quaternary ammonium groups are described especially in FR 1 492 597, and mention may be made of those having the INCI name Polyquaternium-10 and more particularly the polymers sold under the name Ucare Polymer "JR" (JR 400 LT, JR 125 and JR 30M) or "LR" (LR 400 and LR 30M) by the company Amerchol. These polymers are also defined in the CTFA dictionary as quaternary ammoniums of hydroxyethylcellulose that have reacted with an epoxide substituted with a trimethylammonium group.

Cationic cellulose copolymers or cellulose derivatives grafted with a water-soluble quaternary ammonium monomer are described in particular in patent US 4 131 576, and mention may be made of hydroxyalkyl celluloses, for instance hydroxymethyl, hydroxyethyl or hydroxypropyl celluloses grafted, in particular, with a methacryloylethyltrimethylammonium, methacrylamidopropyltrimethylammonium or dimethyldiallylammonium salt. The commercial products corresponding to this definition are more particularly the products sold under the names Celquat L 200 and Celquat H 100 by the company National Starch.

The cationic galactomannan gums are described more particularly in patents US 3 589 578 and US 4 031 307, and mention may be made of guar gums comprising cationic trialkylammonium groups. Use is made, for example, of guar gums modified with a 2,3-epoxypropyltrimethylammonium salt (for example, a chloride). Such products have, for example, the INCI name guar hydroxypropyltrimonium chloride or hydroxypropyl guar hydroxypropyltrimonium chloride. Such products are in particular sold under the names Jaguar C13 S, Jaguar C 15, Jaguar C 17 and Jaguar C162 by the company Rhodia. A compound that is most particularly preferred is a hydroxypropyl guar hydroxypropyltrimonium chloride sold especially under the name Jaguar C162 by the company Rhodia.

Preferably, the cationic polysaccharide(s) are chosen from cationic galactomannan gums, better still from guar gums comprising trialkylammonium, especially trimethylammonium, cationic groups.

According to a preferred embodiment of the invention, the cationic polysaccharide(s) are chosen from guar gums comprising trialkylammonium, especially trimethylammonium, cationic groups, such as the compounds having the INCI name guar hydroxypropyltrimonium chloride or hydroxypropyl guar hydroxypropyltrimonium chloride.

Preferably, the cationic polysaccharide(s) are present in the composition according to the invention in a total content of between 0.01% and 6% by weight, more preferentially between 0.05% and 4% by weight, even more preferentially between 0.1% and 3% by weight, even better still between 0.15% and 2% by weight, relative to the total weight of the composition.

### The cationic polymers (iv) of alkyldiallylamine and of dialkyldiallylammonium

The composition according to the present invention comprises at least one cationic polymer chosen from alkyldiallylamine polymers, dialkyldiallylammonium polymers, and mixtures thereof.

Preferably, the alkyldiallylamine polymer(s) and/or the dialkyldiallylammonium polymer(s) have a cationic charge density greater than or equal to 2 milliequivalents/gram (meq/g), better still greater than or equal to 2.5 meq/g, or even greater than or equal to 3 meq/g, in particular ranging from 2 to 20 meq/g, especially from 2.5 to 10 meq/g, or better still from 3 to 8 meq/g.

The cationic charge density of an alkyldiallylamine polymer and/or of a dialkyldiallylammonium polymer corresponds to the number of moles of cationic charges per unit mass of polymer under conditions in which it is totally ionized. It may be determined by calculation if the structure of the polymer is known, i.e. the structure of the monomers constituting the polymer and their molar proportion or weight proportion. It may also be determined experimentally by the Kjeldahl method.

The alkyldiallylamine polymers and/or the dialkyldiallylammonium polymers that may be used preferably have a weight-average molar mass (Mw) of between 500 and 5×10⁶ approximately and preferably between 10³ and 3×10⁶ approximately.

Preferably, the alkyldiallylamine polymers and/or the dialkyldiallylammonium polymers are chosen from alkyldiallylamine cyclopolymers and/or dialkyldiallylammonium cyclopolymers such as homopolymers or copolymers including, as main constituent of the chain, units corresponding to formula (2) or (3): 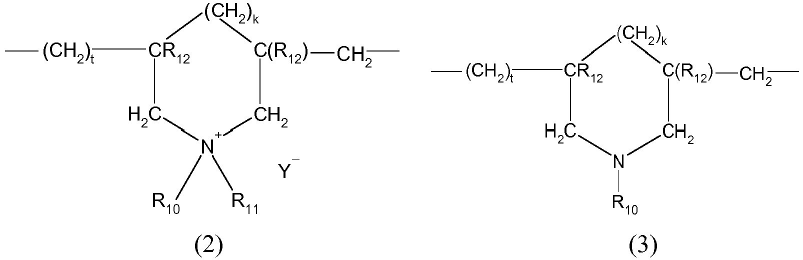 in which
- k and t are equal to 0 or 1, the sum k + t being equal to 1;
- R₁₂ denotes a hydrogen atom or a methyl radical;
- R₁₀ and R₁₁, independently of each other, denote a C₁-C₆ alkyl group, a C₁-C₅ hydroxyalkyl group, a C₁-C₄ amidoalkyl group; or alternatively R₁₀ and R₁₁ may denote, together with the nitrogen atom to which they are attached, a heterocyclic group such as piperidyl or morpholinyl; R₁₀ and R₁₁, independently of each other, preferably denote a C₁-C₄ alkyl group; better still, R₁₀ is a methyl and R₁₁ is a methyl;
- Y⁻ is an anion such as bromide, chloride, acetate, borate, citrate, tartrate, bisulfate, bisulfite, sulfate or phosphate.

More preferentially, mention may be made more particularly of homopolymers of dimethyldiallylammonium salts (for example chloride) such as those having the INCI name Polyquaternium-6, sold, for example, under the name Merquat 100 by the company Nalco, and copolymers of diallyldimethylammonium salts (for example chloride) and of acrylamide such as those having the INCI name Polyquaternium-7, sold especially under the name Merquat 550 or Merquat 7SPR.

According to a preferred embodiment of the invention, the cationic polymer(s) (iv) are chosen from dimethyldiallylammonium chloride homopolymers, copolymers of diallyldimethylammonium chloride and of acrylamide, and mixtures thereof.

More preferentially, the cationic polymer(s) (iv) are chosen from copolymers of diallyldimethylammonium chloride and of acrylamide (of INCI name Polyquaternium-7).

Preferably, the cationic polymer(s) (iv) are present in the composition according to the invention in a total content of between 0.01% and 5% by weight, more preferentially between 0.05% and 3% by weight, even more preferentially between 0.07% and 2% by weight, even better still between 0.08% and 1% by weight, relative to the total weight of the composition.

The weight ratio between the total content of cationic polysaccharide(s) (iii), on the one hand, and the total content of cationic polymer(s) (iv), on the other hand, is greater than or equal to 1.0, preferably between 1.0 and 10.0 and more preferentially between 1.5 and 5.0.

According to a particular embodiment of the invention, when the composition according to the invention comprises one or more guar gums comprising trialkylammonium cationic groups, the weight ratio between the total content of guar gums comprising trialkylammonium cationic groups, on the one hand, and the total content of cationic polymer(s) (iv), on the other hand, is greater than or equal to 1.0, preferably between 1.0 and 10.0 and more preferentially between 1.5 and 5.0.

### Plant oils

The composition according to the present invention comprises one or more plant oils.

The term "oil" means any fatty substance that is in liquid form at room temperature (25°C) and at atmospheric pressure.

The term "fatty substance" means an organic compound that is insoluble in water at room temperature (25°C) and at atmospheric pressure (1.013×10⁵ Pa) (solubility of less than 5% by weight, preferably less than 1% by weight and even more preferentially less than 0.1% by weight). They have in their structure at least one hydrocarbon-based chain including at least 6 carbon atoms. In addition, the fatty substances are generally soluble in organic solvents under the same temperature and pressure conditions, for instance chloroform, dichloromethane, carbon tetrachloride, ethanol, benzene, toluene, tetrahydrofuran (THF), liquid petroleum jelly or decamethylcyclopentasiloxane.

Preferably, the plant oil(s) present in the composition according to the invention are chosen from hydrocarbon-based oils of plant origin such as phytostearyl esters, such as phytostearyl oleate, phytostearyl isostearate and lauroyl/octyldodecyl/phytostearyl glutanate, for example sold under the name Eldew PS203 by Ajinomoto, triglycerides constituted of fatty acid esters of glycerol, the fatty acids of which may have chain lengths ranging from C₄ to C₂₄, these chains possibly being linear or branched, and saturated or unsaturated; these oils are especially heptanoic or octanoic triglycerides, sweet almond oil, argan oil, avocado oil, groundnut oil, camellia oil, safflower oil, beauty-leaf oil, rapeseed oil, coconut kernel, coriander oil, marrow oil, wheatgerm oil, jojoba oil or liquid jojoba wax, linseed oil, macadamia oil, corn germ oil, hazelnut oil, walnut oil, vemonia oil, apricot kernel oil, olive oil, evening-primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil; or alternatively caprylic/capric acid triglycerides, for instance those sold by the company Stéarinerie Dubois or those sold under the names Miglyol 810^{®}, 812^{®} and 818^{®} by the company Dynamit Nobel, and the refined plant-based perhydrosqualene sold under the name Fitoderm by the company Cognis; the plant-based squalane sold, for example, under the name Squalive by the company Biosynthis.

The term "hydrocarbon-based oil" means an oil mainly containing hydrogen and carbon atoms and possibly oxygen, nitrogen, sulfur and/or phosphorus atoms. A hydrocarbon-based oil does not comprise any silicon atoms.

Plant oils are neither silicone-containing nor oxyethylenated.

According to a preferred embodiment of the invention, the plant oil(s) are chosen from sweet almond oil, argan oil, avocado oil, groundnut oil, camellia oil, safflower oil, beauty-leaf oil, rapeseed oil, coconut kernel oil, coriander oil, marrow oil, wheatgerm oil, jojoba oil or liquid jojoba wax, linseed oil, macadamia oil, corn germ oil, hazelnut oil, walnut oil, vemonia oil, apricot kernel oil, olive oil, evening-primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil, and mixtures thereof; more preferentially from soybean oil, castor oil and coconut (or coconut kernel) oil, and mixtures thereof.

In a particular embodiment of the invention, the composition comprises soybean oil.

The total content of plant oil(s) is greater than or equal to 0.1% by weight, preferably between 0.1% and 3% by weight, more preferentially between 0.1% and 1% by weight and even more preferentially between 0.15% and 0.75% by weight, relative to the total weight of the composition.

### Fatty ethers

The composition according to the present invention comprise at least one fatty ether.

According to the invention, the term "fatty ether" means an aliphatic ether comprising at least one non-oxyalkylenated hydrocarbon-based chain comprising from 6 to 40 carbon atoms.

The fatty ethers used according to the invention may be soluble or insoluble in the compositions according to the invention.

Preferably, the fatty ethers that may be used are insoluble in the composition according to the invention.

The fatty ether(s) are chosen from the compounds of formula (4):

R-O-R' (4)

in which:
R and R', which may be identical or different, represent a linear or branched C₆-C₂₅ alkyl or alkenyl radical.

Preferably, R and R' are chosen such that the fatty ether is liquid at a temperature of less than or equal to 30°C, and at atmospheric pressure.

More preferentially, the radicals R and R', which may be identical or different, represent a linear or branched C₆-C₁₂ alkyl or alkenyl radical.

Even more preferentially, the radicals R and R' represent an identical alkyl radical.

Better still, R and R' represent a C₈ radical.

According to a preferred embodiment of the invention, the ether(s) of formula (4) are dialkyl ethers, more preferentially chosen from di-n-hexyl ether, di-n-heptyl ether, di-n-octyl ether, di-n-nonyl ether, di-n-decyl ether, diisodecyl ether, di-n-dodecyl ether, di-n-tetradecyl ether, di-n-hexadecyl ether, di-n-octadecyl ether, and mixtures thereof.

For the purposes of the present invention, the term "dialkyl ether" means an ether of formula (4) in which R and R' are identical.

The fatty ethers may especially be prepared according to the process described in patent application DE 41 27 230.

According to a particularly preferred embodiment of the invention, the ether is di-n-octyl ether (INCI name: dicaprylyl ether).

Di-n-octyl ether is sold, for example, under the reference Cetiol^{®} OE by the company Cognis (MOF) or Rofetan OE by the company Ecovert Oléochimique.

### Nacreous agents

Preferably, the total content of fatty ether(s) is between 0.01% and 5% by weight, more preferentially between 0.05% and 3% by weight, and even more preferentially between 0.1% and 1% by weight, relative to the total weight of the composition.

In a preferred embodiment, the composition according to the invention comprises one or more fatty ethers of formula (4) that are liquid at a temperature equal to 30°C and at atmospheric pressure.

The composition according to the present invention may optionally also comprise at least one nacreous agent.

The term "nacreous agent" means any compound that is capable of giving the composition according to the invention an irisated, iridescent, moiré or metallized appearance or effect.

When the composition according to the invention also comprises at least one fatty ether and at least one nacreous agent, it is understood that the nacreous agent(s) are different from the fatty ether(s) according to the invention.

Preferably, the nacreous agent(s) may be chosen from:
a) esters of polyols containing at least two carbon atoms and of long-chain fatty acids, which are preferentially C₁₀-C₃₀ and even more preferentially C₁₆-C₂₂; such as monoesters or diesters of polyols and of fatty acids; preferably, the polyols are ethylene glycol and polyalkylene glycols containing from 2 to 10 ethylene oxide units;
b) esters of long-chain (C₁₀-C₃₀) monoalcohols, such as cetyl palmitate;
c) ethers of long-chain fatty alcohols that are solid at a temperature of less than or equal to about 30°C and at atmospheric pressure, for instance the dialkyl ethers of formula (I'):

   R-O-R' (I')

   in which R and R', which may be identical or different, denote a linear or branched, saturated or unsaturated alkyl radical including from 10 to 30 carbon atoms and preferably from 14 to 24 carbon atoms, R and R' being chosen such that the compound of formula (I') is solid at a temperature of less than or equal to 30°C and at atmospheric pressure. More particularly, R and R' denote a stearyl radical. These compounds may especially be prepared according to the process described in patent application DE 41 27 230. A distearyl ether that may be used in the context of the present invention is sold under the name Cutina STE by the company HENKEL;
d) long-chain (C₁₀-C₃₀) esters of long-chain (C₁₀-C₃₀) alkanolamides, such as stearamide distearate diethanolamide or stearamide stearate monoethanolamide;
e) single-chain fatty alcohols containing at least 20 carbon atoms, such as behenyl alcohol; and
f) compounds containing from 27 to 48 carbon atoms and including one or two ether and/or thioether or sulfoxide groups, and more particularly corresponding to formula (II'):

   Rₐ-X[C₂H₃(OH)]-CH₂-Y-R_{b} (II')

   in which Rₐ and R_{b} denote, independently of each other, linear C₁₂ to C₂₄ groups;
   X denotes an oxygen atom, a sulfur atom or a sulfoxide or methylene group;
   Y denotes an oxygen atom, a sulfur atom or a sulfoxide or methylene group;
   Rₐ and R_{b} have a value ranging from 24 to 44 and preferably from 28 to 40 inclusive; when X or Y denotes a sulfoxide group, X or Y does not denote a sulfur atom.
   The compounds of formula (II) that are preferably used in accordance with the invention are those for which X denotes an oxygen atom, Y denotes a methylene group and Rₐ and R_{b} denote radicals containing 12 to 22 carbon atoms, these compounds being able to be prepared according to patent EP457688; and
g) coated or uncoated titanium oxides, micas and titanium micas;
h) cyclodextrins and in particular β-cyclodextrin.

According to a preferred embodiment of the invention, the nacreous agent(s) are chosen from esters of polyols containing at least two carbon atoms and of long-chain fatty acids - more preferentially C₁₀-C₃₀ and even more preferentially C₁₆-C₂₂ long-chain fatty acids -; such as monoesters or diesters of polyols and of fatty acids, and mixtures thereof; preferably, the polyols are ethylene glycol and polyalkylene glycols containing from 2 to 10 ethylene oxide units.

Preferably, when the nacreous agent(s) are present in the composition according to the invention, the total content of nacreous agents is between 0.001% and 6% by weight, more preferentially between 0.05% and 5% by weight, and even more preferentially between 0.1% and 3% by weight, relative to the total weight of the composition.

### Nonionic surfactants

The composition according to the present invention may optionally also comprise at least one nonionic surfactant.

In particular, the nonionic surfactants may be chosen from:
- alcohols, α-diols and (C₁₋₂₀)alkylphenols, these compounds being polyethoxylated and/or polypropoxylated and/or polyglycerolated, the number of ethylene oxide and/or propylene oxide groups possibly ranging from 1 to 100, and the number of glycerol groups possibly ranging from 2 to 30; or else these compounds comprising at least one fatty chain including from 8 to 40 carbon atoms and in particular from 16 to 30 carbon atoms; in particular, oxyethylenated alcohols comprising at least one saturated or unsaturated, linear or branched C₈ to C₄₀ alkyl chain, comprising from 1 to 100 mol of ethylene oxide, preferably from 2 to 50 and more particularly from 2 to 40 mol of ethylene oxide and including one or two fatty chains;
- condensates of ethylene oxide and propylene oxide with fatty alcohols;
- polyethoxylated fatty amides preferably containing from 2 to 30 ethylene oxide units, polyglycerolated fatty amides including on average from 1 to 5 and in particular from 1.5 to 4 glycerol groups;
- ethoxylated fatty acid esters of sorbitan, preferably containing from 2 to 40 ethylene oxide units;
- fatty acid esters of saccharose;
- polyoxyalkylenated, preferably polyoxyethylenated, fatty acid esters containing from 2 to 150 mol of ethylene oxide, including oxyethylenated plant oils;
- N-(C₆-C₂₄ alkyl)glucamine derivatives;
- amine oxides such as (C₁₀-C₁₄ alkyl)amine oxides or N-(C₁₀-C₁₄ acyl)aminopropylmorpholine oxides.

Mention may also be made of nonionic surfactants of alkyl(poly)glycoside type, represented especially by the following general formula:

R₁O-(R₂O)ₜ-(G)ᵥ

in which:
- R₁ represents a linear or branched alkyl or alkenyl radical including 6 to 24 carbon atoms and especially 8 to 18 carbon atoms, or an alkylphenyl radical of which the linear or branched alkyl radical includes 6 to 24 carbon atoms and especially 8 to 18 carbon atoms;
- R₂ represents an alkylene radical including 2 to 4 carbon atoms,
- G represents a sugar unit including 5 to 6 carbon atoms,
- t denotes a value ranging from 0 to 10 and preferably from 0 to 4,
- v denotes a value ranging from 1 to 15 and preferably from 1 to 4.

Preferably, the alkyl(poly)glycoside surfactants are compounds of the formula described above in which:
- R₁ denotes a linear or branched, saturated or unsaturated alkyl radical including from 8 to 18 carbon atoms,
- R₂ represents an alkylene radical including 2 to 4 carbon atoms,
- t denotes a value ranging from 0 to 3 and preferably equal to 0,
- G denotes glucose, fructose or galactose, preferably glucose;
- the degree of polymerization, i.e. the value of v, possibly ranging from 1 to 15 and preferably from 1 to 4; the mean degree of polymerization more particularly being between 1 and 2.

The glucoside bonds between the sugar units are generally of 1-6 or 1-4 type and preferably of 1-4 type. Preferably, the alkyl(poly)glycoside surfactant is an alkyl(poly)glucoside surfactant. C₈/C₁₆-Alkyl(poly)glucosides 1,4, and in particular decyl glucosides and caprylyl/capryl glucosides, are most particularly preferred.

Among commercial products, mention may be made of the products sold by the company Cognis under the names Plantaren^{®} (600 CS/U, 1200 and 2000) or Plantacare^{®} (818, 1200 and 2000); the products sold by the company SEPPIC under the names Oramix CG 110 and Oramix^{®} NS 10; the products sold by the company BASF under the name Lutensol GD 70, or else the products sold by the company Chem Y under the name AG10 LK.

Preferably, use is made of C₈/C₁₆-alkyl(poly)glycosides 1,4, in particular as an aqueous 53% solution, such as those sold by Cognis under the reference Plantacare^{®} 818 UP.

Preferentially, the nonionic surfactants are chosen, alone or as a mixture, from:
- oxyethylenated fatty alcohols including at least one saturated or unsaturated, linear or branched, C₈ to C₄₀, especially C₈-C₂₀ and better still C₁₀-C₁₈ alkyl chain, and comprising from 1 to 100 mol of ethylene oxide, preferably from 2 to 50, more particularly from 2 to 40 mol, or even from 3 to 20 mol of ethylene oxide; especially lauryl alcohol containing 4 mol of ethylene oxide (INCI name: Laureth-4) and lauryl alcohol containing 12 mol of ethylene oxide (INCI name: Laureth-12);
- (C₆-C₂₄ alkyl)(poly)glycosides, and more particularly (C₈-C₁₈ alkyl)(poly)glycosides; and
- polyoxyalkylenated, preferably polyoxyethylenated, fatty acid esters containing from 2 to 150 mol of ethylene oxide, including oxyethylenated plant oils.

Preferably, when the nonionic surfactant(s) are present in the composition according to the invention, the total content of nonionic surfactant(s) is between 0.1% and 15% by weight, more preferentially between 0.5% and 10% by weight, even more preferentially between 0.8% and 8% by weight and better still between 1% and 5% by weight, relative to the total weight of the composition.

The composition may comprise a cosmetically acceptable medium. The cosmetically acceptable medium that may be used in the compositions of the invention may comprise water, one or more organic solvents, or a mixture thereof.

Preferably, the composition according to the invention is aqueous.

According to this preference, the amount of water may range from 5% to 98% by weight, better from 25% by weight to 95% by weight, better still from 50% to 90% by weight and even more preferentially from 65% to 85% by weight, relative to the total weight of the composition.

Examples of organic solvents that may especially be used include those that are liquid at 25°C and 1.013 × 10⁵ Pa and that are especially water-soluble, such as C₁-C₇ alcohols, especially C₁-C₇ aliphatic or aromatic monoalcohols, and C₃-C₇ polyols and C₃-C₇ polyol ethers, which may thus be used alone or as a mixture with water. Advantageously, the organic solvent may be chosen from ethanol, isopropanol, propylene glycol, hexylene glycol and glycerol, and mixtures thereof.

Preferably, the pH of the composition is between 3 and 7 and especially between 4 and 6.

The pH of these compositions may be adjusted to the desired value by means of basifying agents or acidifying agents that are usually used. Among the basifying agents, examples that may be mentioned include aqueous ammonia, alkanolamines, and mineral or organic hydroxides. Among the acidifying agents, examples that may be mentioned include mineral or organic acids, for instance hydrochloric acid or orthophosphoric acid, carboxylic acids, for instance acetic acid, tartaric acid, citric acid and lactic acid, and sulfonic acids.

The composition according to the invention may also contain additives used in cosmetics, such as thickeners, preserving agents, fragrances and colourants.

These additives may be present in the composition according to the invention in an amount ranging from 0 to 20% by weight, relative to the total weight of the composition.

A person skilled in the art will take care to select these optional additives and amounts thereof so that they do not harm the properties of the compositions of the present invention.

Preferably, the composition according to the invention is in the form of a shampoo.

Another subject of the invention also relates to the use of the composition as defined previously, for the cosmetic treatment of, more preferentially for washing and/or caring for, keratin materials, especially keratin fibres, in particular human keratin fibres such as the hair.

The composition may be used on wet or dry hair, in rinse-out or leave-in mode.

A subject of the invention is also a process for the cosmetic treatment of, preferably for washing and/or caring for, keratin materials, especially keratin fibres, in particular human keratin fibres such as the hair, comprising at least one step of applying to said keratin materials a composition as defined previously.

According to a preferred embodiment of the invention, a step of rinsing the keratin fibres is performed after the step(s) of applying a composition according to the invention to said fibres.

A step of drying the keratin fibres may be envisaged in the process according to the invention, especially using a heating means such as a hair dryer, a straightening iron, a steam iron or a heating hood, the heating means possibly heating to a temperature ranging from 150°C to 230°C, preferably from 200°C to 220°C.

The examples that follow serve to illustrate the invention without, however, being limiting in nature.

### EXAMPLE:

### Example 1:

Shampoo compositions according to the invention (Inv) and comparative compositions (Comp) were prepared (amounts expressed as weight percentages of active material).

| | A1 (Inv) | A2 (Inv) | A3 (Comp) | A4 (Comp) |
|---|---|---|---|---|
| Sodium laureth sulfate | 14 | 9.8 | 14 | 14 |
| Cocoylbetaine | 5 | 3.47 | 5 | 5 |
| Hydroxypropyl guar hydroxypropyltrimonium chloride | 0.3 | 0.2 | 0.4 | - |
| Polyquaternium-7 | 0.1 | 0.1 | - | 0.4 |
| Soybean oil | 0.25 | 0.175 | 0.25 | 0.25 |
| Carbomer | 0.2 | 0.2 | 0.2 | 0.2 |
| Ethylene glycol distearate | 2 | 2 | 2 | 2 |
| PEG-60 hydrogenated castor oil | 0.75 | 0.5 | 0.75 | 0.75 |
| PEG-55 propylene glycol oleate | 0.2 | 0.2 | 0.2 | 0.2 |
| Dicaprylyl ether | 0.5 | 0.35 | 0.5 | 0.5 |
| PPG-5-Ceteth-20 | - | 0.5 | - | - |
| Sodium chloride | 1.1 | 0.75 | 1.1 | 1.1 |
| Hexylene glycol | 0.5 | - | 0.5 | 0.5 |
| Propylene glycol | 0.2 | 0.2 | 0.2 | 0.2 |
| Glycerol | 2 | - | 2 | 2 |
| Preserving agents | qs | qs | qs | qs |
| pH agent | qs pH 5.3 | qs pH 5.3 | qs pH 5.3 | qs pH 5.3 |
| Water | qs 100 | qs 100 | qs 100 | qs 100 |

Each of the compositions was applied to locks of moderately sensitized pre-moistened hair, at a rate of 0.4 g of composition per gram of hair. The hair is massaged for 15 seconds between the fingers and then rinsed with warm water for 10 seconds, and then drained dry between two fingers.

The properties in terms of disentangling and of smooth feel on locks of wet hair were evaluated by six experts on a scale ranging from 0 (very poor) to 5 (very good) in increments of 0.5.

The averages of the scores obtained are given below:

| | A1 (invention) | A3 (comparative) | A4 (comparative) |
|---|---|---|---|
| Disentangling of wet hair | 3.5 | 2.5 | 2 |
| Smooth feel of wet hair | 4 | 3.5 | 2.5 |

It is found that, for all the evaluations performed, composition A1 according to the invention was judged to be superior to the comparative compositions A3 and A4 in terms of disentangling and of smooth feel on wet hair.

### Example 2:

Composition B1 according to the invention (Inv) and comparative composition B2 (Comp) were prepared (amounts expressed as weight percentages of active material).

| | **B1 (Inv)** | **B2 (Inv)** |
|---|---|---|
| Sodium laureth sulfate | 9.8 | |
| Cocoylbetaine | 3.47 | |
| Hydroxypropyl guar hydroxypropyltrimonium chloride | 0.2 | |
| Polyquaternium-7 | 0.1 | |
| Soybean oil | **0.175** | **0.05** |
| Carbomer | 0.2 | |
| Ethylene glycol distearate | 2 | |
| PEG-60 hydrogenated castor oil | 0.5 | |
| PEG-55 propylene glycol oleate | 0.2 | |
| Dicaprylyl ether | 0.35 | |
| PPG-5-Ceteth-20 | 0.5 | |
| Sodium chloride | 0.75 | |
| Propylene glycol | 0.2 | |
| Preserving agents | Qs | |
| pH agent | Qs pH 5.3 | |
| Water | Qs 100 | |

The compositions B1 and B2 are each applied to a half-head of natural brown hair, and whose hair has been previously washed with a standard shampoo.

Compositions B1 and B2 were applied at a rate of 6g per half-head, distributed homogeneously from the roots to the tips.

The hair is massaged for 15 seconds between the fingers and then rinsed with warm water (35°C) for 10 seconds, and then wrung out.

The wet hair is then evaluated, and once the assessment on wet hair is complete, the hair is dried with a hair dryer and disentangled in order to be evaluated again, dry.

To evaluate the impact of the invention on cosmetic performance, six experts took into account several criteria: smoothness and lightness for wet hair, homogeneity and softness for dry hair.

The performances were evaluated during a blind test: each of the 6 experts pronounced:
- *for wet hair:* on the side of the head which presented the most lightness and the most smooth touch (noted "+"), as well as
- *for dry hair:* on the side of the head which had the softest and most homogenous feel (noted "+").

Therefore, the sides of the head that had the least lightness and the least smooth touch (for wet hair), and the least soft and least homogeneous feel (for dry hair), are noted "-".

### Gestures

To evaluate the smoothness, the softness and the homogeneous feel, the expert grasps a lock of hair between thumb and forefinger and slides his fingers along the lock from the top to the ends.

For the lightness, the expert raises the hair in big locks with the hands and looks at the way the hair falls: the light hair is individualized, fluid and does not fall in packets.

The results are summarized in the tables below.

| **Wet hair** | Smoothness | | Lightness | |
|---|---|---|---|---|
| | **B1** (Inv) | **B2** (Comp) | **B1** (Inv) | **B2** (Comp) |
| Expert 1 | + | - | + | - |
| Expert 2 | + | - | + | - |
| Expert 3 | + | - | + | - |
| Expert 4 | + | - | + | - |
| Expert 5 | + | - | + | - |
| Expert 6 | + | - | + | - |

| **Dry hair** | Softness | | Homogeneous feel | |
|---|---|---|---|---|
| | **B1** (Inv) | **B2** (Comp) | **B1** (Inv) | **B2** (Comp) |
| Expert 1 | + | - | + | - |
| Expert 2 | + | - | + | - |
| Expert 3 | + | - | + | - |
| Expert 4 | + | - | + | - |
| Expert 5 | + | - | + | - |
| Expert 6 | + | - | + | - |

On wet hair, 6 out of 6 (100%) experts judged that the fibres treated with the composition B1 according to the invention comprising more than 0.1% by weight of plant oil relative to the total weight of the composition, were smoother and lighter than those treated with the comparative composition B2 comprising less than 0.1% by weight of plant oil relative to the total weight of the composition.

On dry hair, 6 out of 6 (100%) experts judged that the fibres treated with the composition B1 according to the invention comprising more than 0.1% by weight of plant oil relative to the total weight of the composition, were softer and more homogeneous than those treated with the comparative composition B2 comprising less than 0.1% by weight of plant oil relative to the total weight of the composition.

## Claims

1. Composition comprising:
(i) at least one anionic surfactant,
(ii) at least one amphoteric or zwitterionic surfactant,
(iii) at least one cationic polymer chosen from cationic polysaccharides,
(iv) at least one cationic polymer chosen from alkyldiallylamine polymers, dialkyldiallylammonium polymers, and mixtures thereof,
(v) one or more plant oils, the total content of which is greater than or equal to 0.1% by weight relative to the total weight of the composition, and
(vi) at least one fatty ether chosen from the compounds of formula (4) below:
R-O-R' (4)
in which:
R and R', which may be identical or different, represent a linear or branched C₆-C₂₅ alkyl or alkenyl radical;
it being understood that the weight ratio between the total content of cationic polysaccharide(s) (iii), on the one hand, and the total content of cationic polymer(s) (iv), on the other hand, is greater than or equal to 1.0.

2. Composition according to the preceding claim, which is silicone-free.

3. Composition according to either of the preceding claims, **characterized in that** the anionic surfactant(s) are chosen from:
- C₆-C₂₄ and especially C₁₂-C₂₀ alkyl sulfates;
- C₆-C₂₄ and especially C₁₂-C₂₀ alkyl ether sulfates, preferably comprising from 1 to 20 ethylene oxide units;
- C₆-C₂₄ and especially C₁₂-C₂₀ alkyl sulfosuccinates, especially lauryl sulfosuccinates;
- C₆-C₂₄ and especially C₁₂-C₂₀ alkyl ether sulfosuccinates;
- (C₆-C₂₄)acylisethionates, preferably (C₁₂-C₁₈)acylisethionates;
- C₆-C₂₄ and especially C₁₂-C₂₀ acylsarcosinates; especially palmitoylsarcosinates;
- (C₆-C₂₄)alkyl ether carboxylates, preferably (C₁₂-C₂₀)alkyl ether carboxylates;
- polyoxyalkylenated (C₆-C₂₄)alkyl(amido) ether carboxylic acids and salts thereof, in particular those including from 2 to 50 alkylene oxide and in particular ethylene oxide groups;
- C₆-C₂₄ and especially C₁₂-C₂₀ acylglutamates;
- C₆-C₂₄ and especially C₁₂ -C₂₀ acylglycinates;
- and also salts thereof, in particular the alkali metal or alkaline-earth metal or zinc, ammonium or amino alcohol salts thereof;
- and mixtures thereof.

4. Composition according to any one of the preceding claims, **characterized in that** the amphoteric or zwitterionic surfactant(s) are chosen from (C₈-C₂₀)alkylbetaines such as cocoylbetaine, (C₈-C₂₀)alkylamido(C₃-C₈)alkylbetaines such as cocamidopropylbetaine, and mixtures thereof.

5. Composition according to any one of the preceding claims, **characterized in that** the weight ratio between the total content of anionic surfactant(s), on the one hand, and the total content of amphoteric or zwitterionic surfactant(s), on the other hand, is between 1.0 and 4.0, preferably between 1.0 and 3.5 and more preferentially between 1.5 and 3.0.

6. Composition according to any one of the preceding claims, **characterized in that** the cationic polysaccharide(s) are chosen from cationic celluloses and cationic galactomannan gums; preferably from cationic galactomannan gums, more preferentially from guar gums comprising trialkylammonium, especially trimethylammonium, cationic groups.

7. Composition according to any one of the preceding claims, **characterized in that** the total content of cationic polysaccharide(s) is between 0.01% and 6% by weight, more preferentially between 0.05% and 4% by weight, even more preferentially between 0.1% and 3% by weight, and better still between 0.15% and 2% by weight, relative to the total weight of the composition.

8. Composition according to any one of the preceding claims, **characterized in that** the cationic polymer(s) (iv) are chosen from dimethyldiallylammonium chloride homopolymers, copolymers of diallyldimethylammonium chloride and of acrylamide, and mixtures thereof; preferably from copolymers of diallyldimethylammonium chloride and of acrylamide.

9. Composition according to any one of the preceding claims, **characterized in that** the total content of cationic polymer(s) (iv) is between 0.01 % and 5% by weight, more preferentially between 0.05% and 3% by weight, even more preferentially between 0.07% and 2% by weight, and better still between 0.08% and 1% by weight, relative to the total weight of the composition.

10. Composition according to any one of the preceding claims, **characterized in that** the weight ratio between the total content of cationic polysaccharide(s) (iii), on the one hand, and the total content of cationic polymer(s) (iv), on the other hand, is between 1.0 and 10.0, preferably between 1.5 and 5.0.

11. Composition according to any one of the preceding claims, **characterized in that** the plant oil(s) are chosen from sweet almond oil, argan oil, avocado oil, groundnut oil, camellia oil, safflower oil, beauty-leaf oil, rapeseed oil, coconut kernel oil, coriander oil, marrow oil, wheatgerm oil, jojoba oil or liquid jojoba wax, linseed oil, macadamia oil, corn germ oil, hazelnut oil, walnut oil, vernonia oil, apricot kernel oil, olive oil, evening-primrose oil, palm oil, passion flower oil, grapeseed oil, rose oil, castor oil, rye oil, sesame oil, rice bran oil, camelina oil, soybean oil, sunflower oil, pracaxi oil, babassu oil, mongongo oil, marula oil, arara oil, shea butter oil, Brazil nut oil, and mixtures thereof; more preferentially from soybean oil, castor oil and coconut (or coconut kernel) oil, and mixtures thereof; even more preferentially from soybean oil.

12. Composition according to any one of the preceding claims, **characterized in that** the total content of plant oil(s) is between 0.1% and 3% by weight, more preferentially between 0.1% and 1% by weight, even more preferentially between 0.15% and 0.75% by weight, relative to the total weight of the composition.

13. Composition according to any one of the preceding claims, **characterized in that** the radicals R and R' of said formula (4) are chosen such that the fatty ether is liquid at a temperature of less than or equal to 30°C and at atmospheric pressure.

14. Use of the composition as defined according to any one of Claims 1 to 13, for the cosmetic treatment of, preferably for washing and/or caring for, keratin materials, especially keratin fibres, in particular human keratin fibres such as the hair.

15. Process for the cosmetic treatment of, preferably for washing and/or caring for, keratin materials, especially keratin fibres, in particular human keratin fibres such as the hair, comprising at least one step of applying to said keratin materials a composition as defined according to any one of Claims 1 to 13.

## Patentansprüche

1. Zusammensetzung, umfassend:
(i) wenigstens ein anionisches Tensid,
(ii) wenigstens ein amphoteres oder zwitterionisches Tensid,
(iii) wenigstens ein kationisches Polymer ausgewählt aus kationischen Polysacchariden,
(iv) wenigstens ein kationisches Polymer ausgewählt aus Alkyldiallylaminpolymeren, Dialkyldiallylammoniumpolymeren und Gemischen davon, und
(v) ein oder mehrere Pflanzenöle, deren Gesamtgehalt größer als oder gleich 0,1 Gew.-%, bezogen auf das Gesamtgewicht der Zusammensetzung, ist; und
(vi) wenigstens einen Fettether ausgewählt aus den Verbindungen der nachstehenden Formel (4):
R-O-R' (4)
wobei:
R und R', die gleich oder verschieden sein können, einen linearen oder verzweigten C₆-C₂₅ Alkyl- oder Alkenylrest darstellen;
mit der Maßgabe, dass das Gewichtsverhältnis zwischen dem Gesamtgehalt an kationischen Polysaccharid(en) (iii) einerseits und dem Gesamtgehalt an kationischen Polymer(en) (iv) andererseits größer als oder gleich 1,0 ist.

2. Zusammensetzung gemäß dem vorstehenden Anspruch, die siliconfrei ist.

3. Zusammensetzung gemäß einem der vorstehenden Ansprüche, **dadurch gekennzeichnet, dass** die anionischen Tensid(e) ausgewählt sind aus:
- C₆-C₂₄- und insbesondere C₁₂-C₂₀Alkylsulfaten;
- C₆-C₂₄- und insbesondere C₁₂-C₂₀Alkylethersulfaten, vorzugsweise umfassend von 1 bis 20 Ethylenoxideinheiten;
- C₆-C₂₄- und insbesondere C₁₂-C₂₀Alkylsulfosuccinaten, insbesondere Laurylsulfosuccinaten;
- C₆-C₂₄- und insbesondere C₁₂-C₂₀Alkylethersulfosuccinaten;
- (C₆-C₂₄) Acylisethionaten, vorzugsweise (C₁₂-C₁₈) Acylisethionaten;
- C₆-C₂₄- und insbesondere C₁₂-C₂₀Acylsarcosinaten; insbesondere Palmitoylsarcosinaten;
- (C₆-C₂₄) Alkylethercarboxylaten, vorzugsweise (C₁₂-C₂₀) Alkylethercarboxylaten;
- polyoxyalkylenierten (C₆-C₂₄)Alkyl(amido)ethercarbonsäuren und Salzen davon, insbesondere jene, die von 2 bis 50 Alkylenoxidgruppen, insbesondere Ethylenoxidgruppen, enthalten;
- C₆-C₂₄- und insbesondere C₁₂-C₂₀Acylglutamaten;
- C₆-C₂₄- und insbesondere C₁₂-C₂₀Acylglycinaten;
- und auch Salzen davon, insbesondere den Alkalimetall- oder Erdalkalimetall- oder Zink-, Ammonium- oder Aminoalkoholsalzen davon;
- und Gemischen davon.

4. Zusammensetzung gemäß einem der vorstehenden Ansprüche, **dadurch gekennzeichnet, dass** die amphoteren oder zwitterionischen Tensid(e) ausgewählt sind aus (C₈-C₂₀) Alkylbetainen, wie z. B. Cocoylbetain, (C₈-C₂₀) Alkylamido (C₃-C₈) alkylbetainen, wie z. B. Cocamidopropylbetain, und Gemischen davon.

5. Zusammensetzung gemäß einem der vorstehenden Ansprüche, **dadurch gekennzeichnet, dass** das Gewichtsverhältnis zwischen dem Gesamtgehalt an anionischen Tensid(en) einerseits und dem Gesamtgehalt an amphoteren oder zwitterionischen Tensid(en) andererseits zwischen 1,0 und 4,0, vorzugsweise zwischen 1,0 und 3, 5 und bevorzugter zwischen 1,5 und 3,0 beträgt.

6. Zusammensetzung gemäß einem der vorstehenden Ansprüche, **dadurch gekennzeichnet, dass** die kationischen Polysaccharid(e) ausgewählt sind aus kationischen Cellulosen und kationischen Galactomannangummis; vorzugsweise aus kationischen Galactomannangummis, bevorzugter aus Guargummis, die kationische Trialkylammonium-, insbesondere Trimethylammoniumgruppen, umfassen.

7. Zusammensetzung gemäß einem der vorstehenden Ansprüche, **dadurch gekennzeichnet, dass** der Gesamtgehalt an kationischen Polysaccharid(en) zwischen 0,01 Gew.-% und 6 Gew.-%, bevorzugter zwischen 0,05 Gew.-% und 4 Gew.-%, noch bevorzugter zwischen 0,1 Gew.-% und 3 Gew.-% und sogar noch besser zwischen 0,15 Gew.-% und 2 Gew.-%, bezogen auf das Gesamtgewicht der Zusammensetzung, beträgt.

8. Zusammensetzung gemäß einem der vorstehenden Ansprüche, **dadurch gekennzeichnet, dass** die kationischen Polymer(e) (iv) ausgewählt sind aus Dimethyldiallylammoniumchlorid-Homopolymeren, Copolymeren von Diallyldimethylammoniumchlorid und Acrylamid, und Gemischen davon; vorzugsweise aus Copolymeren von Diallyldimethylammoniumchlorid und Acrylamid.

9. Zusammensetzung gemäß einem der vorstehenden Ansprüche, **dadurch gekennzeichnet, dass** der Gesamtgehalt an kationischen Polymer(en) (iv) zwischen 0,01 Gew.-% und 5 Gew.-%, bevorzugter zwischen 0,05 Gew.-% und 3 Gew.-%, noch bevorzugter zwischen 0,07 Gew.-% und 2 Gew.-% und sogar noch besser zwischen 0,08 Gew.-% und 1 Gew.-%, bezogen auf das Gesamtgewicht der Zusammensetzung, beträgt.

10. Zusammensetzung gemäß einem der vorstehenden Ansprüche, **dadurch gekennzeichnet, dass** das Gewichtsverhältnis zwischen dem Gesamtgehalt an kationischen Polysaccharid(en) (iii) einerseits und dem Gesamtgehalt an kationischen Polymer(en) (iv) andererseits zwischen 1,0 und 10,0, vorzugsweise zwischen 1,5 und 5,0, beträgt.

11. Zusammensetzung gemäß einem der vorstehenden Ansprüche, **dadurch gekennzeichnet, dass** die Pflanzenöl(e) ausgewählt sind aus Süßmandelöl, Arganöl, Avocadoöl, Erdnussöl, Kamelienöl, Distelöl, Schönblattöl, Rapsöl, Kokosnusskernöl, Korianderöl, Kürbisöl, Weizenkeimöl, Jojobaöl oder flüssigem Jojobawachs, Leinsamenöl, Macadamiaöl, Maiskeimöl, Haselnussöl, Walnussöl, Vernoniaöl, Aprikosenkernöl, Olivenöl, Nachtkerzenöl, Palmöl, Passionsblumenöl, Traubenkernöl, Rosenöl, Rizinusöl, Roggenöl, Sesamöl, Reiskleieöl, Leindotteröl, Sojabohnenöl, Sonnenblumenöl, Pracaxiöl, Babassuöl, Mongongoöl, Marulaöl, Araraöl, Sheabutteröl, Paranussöl und Gemischen davon; bevorzugter aus Sojabohnenöl, Rizinusöl und Kokosnussöl (oder Kokosnusskernöl) und Gemischen davon; noch bevorzugter aus Sojabohnenöl.

12. Zusammensetzung gemäß einem der vorstehenden Ansprüche, **dadurch gekennzeichnet, dass** der Gesamtgehalt an Pflanzenöl(en) zwischen 0,1 Gew.-% und 3 Gew.-%, bevorzugter zwischen 0,1 Gew.-% und 1 Gew.-%, noch bevorzugter zwischen 0,15 Gew.-% und 0,75 Gew.-%, bezogen auf das Gesamtgewicht der Zusammensetzung, beträgt.

13. Zusammensetzung gemäß einem der vorstehenden Ansprüche, **dadurch gekennzeichnet, dass** die Reste R und R' der Formel (4) so ausgewählt sind, dass der Fettether bei einer Temperatur von kleiner als oder gleich 30°C und atmosphärischem Druck flüssig ist.

14. Verwendung der Zusammensetzung gemäß einem der Ansprüche 1 bis 13 zur kosmetischen Behandlung von, vorzugsweise zum Waschen und/oder Pflegen von, Keratinmaterialien, insbesondere Keratinfasern, insbesondere menschlichen Keratinfasern, wie z. B. dem Haar.

15. Verfahren zur kosmetischen Behandlung von, vorzugsweise zum Waschen und/oder Pflegen von, Keratinmaterialien, insbesondere Keratinfasern, insbesondere menschlichen Keratinfasern, wie z. B. dem Haar, umfassend wenigstens einen Schritt des Aufbringens einer Zusammensetzung gemäß einem der Ansprüche 1 bis 13 auf die Keratinmaterialien.

## Revendications

1. Composition comprenant :
(i) au moins un tensioactif anionique,
(ii) au moins un tensioactif amphotère ou zwittérionique,
(iii) au moins un polymère cationique choisi parmi les polysaccharides cationiques,
(iv) au moins un polymère cationique choisi parmi les polymères d'alkyl diallyl amine, les polymères de dialkyl diallyl ammonium, et leurs mélanges,
(v) une ou plusieurs huiles végétales, dont la teneur totale est supérieure ou égale à 0,1% en poids par rapport au poids total de la composition, et
(vi) au moins un éther gras choisi parmi les composés de formule (4) ci-dessous :
R-O-R' (4)
dans laquelle :
R et R', identiques ou différents, représentent un radical alkyle ou alcényle en C₆-C₂₅, linéaire ou ramifié ;
étant entendu que le rapport pondéral entre la teneur totale en polysaccharide(s) cationique(s) (iii) d'une part et la teneur totale en polymère(s) cationique(s) (iv) d'autre part, est supérieur ou égale à 1,0.

2. Composition selon la revendication précédente, dépourvue de silicone.

3. Composition selon l'une quelconque des revendications précédentes, **caractérisée en ce que** le ou les tensioactifs anioniques sont choisis parmi :
- les alkylsulfates en C₆-C₂₄, notamment en C₁₂-C₂₀ ;
- les alkyléthersulfates en C₆-C₂₄, notamment en C₁₂-C₂₀, comprenant de préférence de 1 à 20 motifs oxyde d'éthylène,
- les alkylsulfosuccinates en C₆-C₂₄, notamment en C₁₂-C₂₀, notamment les laurylsulfosuccinates ;
- les alkyléthersulfosuccinates en C₆-C₂₄, notamment en C₁₂-C₂₀ ;
- les (C₆-C₂₄)acyliséthionates, de préférence les (C₁₂-C₁₈)acyliséthionates ;
- les acylsarcosinates en C₆-C₂₄, notamment en C₁₂-C₂₀ ; notamment les palmitoylsarcosinates ;
- les alkyl(C₆-C₂₄)éthercarboxylates, de préférence les alkyl(C₁₂-C₂₀)éthercarboxylates ;
- les acides alkyl(C₆-C₂₄)(amido)éthercarboxyliques polyoxyalkylénés et leurs sels, en particulier ceux comportant de 2 à 50 groupements oxyde d'alkylène, en particulier d'éthylène ;
- les acylglutamates en C₆-C₂₄, notamment en C₁₂-C₂₀ ;
- les acylglycinates en C₆-C₂₄, notamment en C₁₂-C₂₀ ;
- ainsi que leurs sels, en particulier leurs sels de métaux alcalins ou alcalino-terreux ou de zinc, d'ammonium, ou d'aminoalcool ;
- et leurs mélanges.

4. Composition selon l'une quelconque des revendications précédentes, **caractérisée en ce que** le ou les tensioactifs amphotères ou zwittérioniques sont choisis parmi les alkyl(C₈-C₂₀)bétaïnes telles que la cocobétaïne, les alkyl(C₈-C₂₀)amidoalkyl(C₃-C₂₀)bétaïnes telles que la cocamidopropylbétaïne, et leurs mélanges.

5. Composition selon l'une quelconque des revendications précédentes, **caractérisée en ce que** le rapport pondéral entre la teneur totale en tensioactif(s) anionique(s) d'une part et la teneur totale en tensioactif(s) amphotère(s) ou zwittérionique(s) d'autre part, est compris entre 1,0 et 4,0, de préférence compris entre 1,0 et 3,5, plus préférentiellement compris entre 1,5 et 3,0.

6. Composition selon l'une quelconque des revendications précédentes, **caractérisée en ce que** le ou les polysaccharides cationiques sont choisis parmi les celluloses cationiques et les gommes de galactomannanes cationiques ; de préférence parmi les gommes de galactomannanes cationiques, plus préférentiellement parmi les gommes de guar comprenant des groupements cationiques trialkylammonium, notamment triméthylammonium.

7. Composition selon l'une quelconque des revendications précédentes, **caractérisée en ce que** la teneur totale en polysaccharide(s) cationique(s) est comprise entre 0,01% et 6% en poids, plus préférentiellement comprise entre 0,05% et 4% en poids, plus préférentiellement encore comprise entre 0,1% et 3% en poids, encore mieux comprise entre 0,15% et 2% en poids, par rapport au poids totale de la composition.

8. Composition selon l'une quelconque des revendications précédentes, **caractérisée en ce que** le ou les polymères cationiques (iv) sont choisis parmi les homopolymères de chlorure de diméthyldiallylammonium, les copolymères de chlorure de diallyldiméthylammonium et d'acrylamide, et leurs mélanges ; de préférence parmi les copolymères de chlorure de diallyldiméthylammonium et d'acrylamide.

9. Composition selon l'une quelconque des revendications précédentes, **caractérisée en ce que** la teneur totale en polymère(s) cationique(s) (iv) est comprise entre 0,01% et 5% en poids, plus préférentiellement comprise entre 0,05% et 3% en poids, plus préférentiellement encore comprise entre 0,07% et 2% en poids, encore mieux comprise entre 0,08% et 1% en poids, par rapport au poids totale de la composition.

10. Composition selon l'une quelconque des revendications précédentes, **caractérisée en ce que** le rapport pondéral entre la teneur totale en polysaccharide(s) cationique(s) (iii) d'une part et la teneur totale en polymère(s) cationique(s) (iv) d'autre part, est compris entre 1,0 et 10,0, de préférence compris entre 1,5 et 5,0.

11. Composition selon l'une quelconque des revendications précédentes, **caractérisée en ce que** la ou les huiles végétales sont choisies parmi l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyau d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de cameline, l'huile de soja, l'huile de tournesol, l'huile de pracaxi, l'huile de babassu, l'huile de mongongo, l'huile de marula, l'huile d'arara, l'huile de beurre de karité, l'huile de noix du brésil, et leurs mélanges ; plus préférentiellement parmi l'huile de soja, l'huile de ricin et l'huile de coco (ou de coprah), et leurs mélanges ; plus préférentiellement encore parmi l'huile de soja.

12. Composition selon l'une quelconque des revendications précédentes, **caractérisée en ce que** la teneur totale en huile(s) végétale(s) est comprise entre 0,1% et 3% en poids, plus préférentiellement comprise entre 0,1% et 1% en poids, plus préférentiellement encore comprise entre 0,15% et 0,75% en poids, par rapport au poids total de la composition.

13. Composition selon l'une quelconque des revendications précédentes, caractérisée en que les radicaux R et R' de ladite formule (4) sont choisis de telle sorte que l'éther gras soit liquide à une température inférieure ou égale à 30°C et à pression atmosphérique.

14. Utilisation de la composition telle que définie selon l'une quelconques des revendications 1 à 13, pour le traitement cosmétique, de préférence pour le lavage et/ou le soin, des matières kératiniques, notamment des fibres kératiniques, en particulier des fibres kératiniques humaines telles que les cheveux.

15. Procédé de traitement cosmétique, de préférence de lavage et/ou de soin, des matières kératiniques, notamment des fibres kératiniques, en particulier des fibres kératiniques humaines telles que les cheveux, comprenant au moins une étape d'application sur lesdites matières kératiniques d'une composition telle que définie selon l'une quelconque des revendications 1 à 13.
